# EUROPEAN PATENT APPLICATION

(11) **EP 2 524 965 A1**
(43) Date of publication of application: **21.11.2012**
(21) Application number: 11166266.4
(22) Date of filing: 16.05.2011
(51) Int. Cl.: C12Q 1/56, G01N 33/49, G01N 33/86, G01N 33/52

(54) **Increase in storage lifetime of a thrombin sensor**

(71) Applicant: The Swatch Group Research and Development Ltd., 2074 Marin (CH)
(72) Inventor: Schuhmann, Wolfgang, 44799 Bochum (DE); Frenkel, Erik Jan, 2000 Neuchâtel (CH)
(74) Representative: Ravenel, Thierry Gérard Louis

(57) **Abstract**

A blood coagulation time measuring sensor comprising a surface, a resin layer applied thereto, including at least one substance with a detection function, characterized in that the resin layer comprises a lacquer system, wherein said lacquer system
i) is suitable for immobilization of the at least one substance with a detection function on the surface; and
ii) provides a negatively charged surface layer;
iii) and allows the replacement of biological blood coagulation promoters by non-biological chemically inert and stable compounds.

## Description

### Background of the Invention

This invention relates to a blood coagulation time measuring sensor with increased storage lifetime through replacement of the main biological component by a non-biologically chemically inert and stable synthetic compound.

The invention concerns more particularly a blood coagulation time measuring sensor allowing the Prothrombin time (PT) to be determined by an electroanalytical method such as amperometry when the reagent composition deposited on the sensor includes a chemical substrate of which a terminal fraction can be selectively cut off by the thrombin enzyme by releasing a charged and/or redox active group.

Checking the blood coagulation time, i.e. the aptitude of the blood constituents to form a clot to prevent the risk of haemorrhage, forms part of routine examinations. It is for example necessary during treatment with anticoagulants in the case of heart diseases to be able to adjust the dosage of the medicine accurately in order to avoid the risk of haemorrhages in the event of overdose or conversely, the risk of thrombosis if the quantity of anticoagulant administered is insufficient.

The blood coagulation cascade involves two pathways, an intrinsic and extrinsic pathway. Both pathways are complex and involve numerous different clotting factors. Therefore, to measure the so-called "Quick"-time as a value for the blood coagulation time of patients, often an oral anti-coagulation treatment, complex chemistry in general is needed. This chemistry normally includes substances that provide a negatively charged lipid surface and blood-coagulation promoters like thromboplastins (tissue factors) from biological origin.

Different parameters have been retained for performing this determination, but the most common is measuring the prothrombin time

(PT) after activation, i.e. the period of time after which the formation of a clot is observed with a blood sample taken from the patient. In such *in vitro* measurement systems the clotting time can be shortened by adding an emulsion of negatively-charged phospholipids and "thromboplastin", a saline brain extract containing tissue factor to decrease the measurement time.

Although this type of chemistry is well known to people using and producing those measuring systems, in particular the development of home-care blood-coagulation measuring systems prompted the development of more robust chemistry.

Therefore one objective of present invention is to circumvent the inherent instability of such blood coagulation time measuring sensors which is due to the labile biological components that are involved in the biochemical blood-coagulation cascade.

US 6,352,630 discloses an electrochemical system for measuring a value representing the coagulation time of a drop of whole blood where a specific reagent is immobilized, including at least one chemical substrate one terminal link of which can be cut off by the thrombin enzyme to give redox active groups. Upon digestion of the thrombin substrate by thrombin, the charged groups are released and migrate to a working electrode. The current produced by oxidation or reduction of the released groups is measured at the working electrode and is correlated with a value representing the coagulation time.

Sensor system according to US 6,352,630 requires a large amount of optimization. Especially the use of the biological blood-coagulation promoter requires a defined and stable reaction environment, where the medium is kept at a defined pH and with sufficient ionic strength. Moreover, the biological blood-coagulation promoter has a limited stability especially at variable or elevated temperatures which limits the storage stability of related sensor. In addition, the biological blood-coagulation promoter is obtained from natural sources which lead unequivocally to batch to batch variations in its properties. Thus, optimization and fabrication of reproducible sensors involving biological blood-coagulation promoters remains difficult. US 6,352,630 also discloses that the phospholipid composition of the tissue factor used has an impact on the determination of the prothrombin time. A tissue factor with phospholipids having a reverse PS/PC ratio, in which the quantity of negative phospholipids is greater than the quantity of positive phospholipids, allows a better determination of the signal (signal height and linearity of signal). The drawbacks of such complicated chemistry, i.e. the necessity to have biological compounds as a blood coagulation promoters in the system, are obvious.

Especially the development of home-care systems for the measurement of blood coagulation time of patients requires sensor chemistry to be as robust as possible, so that surveillance of factors like storage periods become less important and storage conditions, such as temperature and humidity, are no more essential. Blood coagulation time measuring systems described in the prior art generally need storage at 4°C and their storage period normally cannot exceed much more than one year. Such storage conditions of course also imply the distribution of such sensors in an uncut cooling chain. Additionally, due to the integration of a biological blood-coagulation promoter during sensor preparation the sensor fabrication protocols have to avoid elevated temperatures, organic solvents, and fast drying. Finally the use of biological materials give rise to an absolute need for individual calibration of lots of sensors during the production process, since biological substances are not identical from one lot to another. In case of the total removal of all material from biological origin, both conditioning requirements for storage and the maximal storage time per se become much less critical.

For the above reasons there was a need for a system that can overcome the drawbacks of the blood coagulation time measurement sensor disclosed in the prior art and that was suitable for use in a home-care system for the measurement of blood coagulation time. The problem to be addressed by present invention was to provide a blood coagulation time measuring sensor with increased storage lifetime.

The immobilization of detection elements on surfaces can be effected by absorption, cross-linking or by inclusion in a polymer film as disclosed in US 3,839,175, Strike et al. Biosens. Bioelectron., 10 (1995) 61-66 and Schuhmann, Mikrochim. Acta, 121 (1995) 1-29, respectively. The detection elements are usually embedded in a matrix that is deposited on the surface either by radical polymerization as for example disclosed in EP-A0691408, lithographic methods as disclosed in McGall et al., J.Am.Chem.Soc., 119 (1997) 5081, or printing methods as disclosed in G.F. Khan, Electroanalysis, 9 (1997) 325-329.

US2003/0153061 discloses the use of the more versatile electro deposition method for immobilizing detection elements on sensor surfaces. It is known from US2003/0153061 that it is possible to use various types of electro-polymerizable lacquers for an electro deposition of layers of the type used as protective layer at the inside of food cans also for the immobilization of enzymes, thereby preserving their enzymatic activity.

Another example for such method is given in Shkotova et al. (2006), Material Science and Engineering, Volume 26, Issues 2-3, 411-414, where an amperometric sensor for ethanol detection is disclosed. The alcohol oxidase is electrochemically deposited on the sensor surface within a resydrol lacquer film. Smutok et al. (2005), Biosensors and Bioelectronics Volume 20, 1285-1290 discloses the electro deposition of flavocytochrome *b*₂ with electro deposition lacquers such as the anodic electro deposition resydrol lacquers. In another example by Ngounou et al. (2004), Electrochimica Acta, Volume 49, 3855-3863 the combinatorial synthesis of a library of acrylic acid-based polymers and their evaluation as immobilisation matrix for amperometric biosensors was disclosed.

In the present invention one objective was to develop a lacquer system as described above allowing the immobilization of an amperogenic thrombin substrate as disclosed in US 6,352,630.

Another objective of the invention is to provide through said lacquer material at the same time the negatively charged surface as mentioned as a requirement for the blood-coagulation cascade to proceed.

### Summary of the invention

The object of the present invention is to overcome the drawbacks which exist in the blood coagulation time measurement systems of the prior art by providing an improved sensor with increased storage lifetime.

The present invention thus concerns a blood coagulation time measuring sensor comprising a surface, a resin layer applied thereto, including at least one substance with a detection function, characterized in that the resin layer comprises a lacquer system, wherein said lacquer system
i) is suitable for immobilization of the at least one substance with a detection function on the surface; and
ii) provides a negatively charged surface layer; and
iii) allows the replacement of biological blood coagulation promoters by integration of non-biological chemically inert and stable synthetic compounds.

According to the invention, an increased storage lifetime is achieved through replacement of the main biological components of the blood coagulation cascade by non-biologically chemically inert and stable compounds. At the same time the measuring time of such system is not significantly prolonged as compared with sensors based on biological compounds which function as blood coagulation promoters.

According to the invention, the resin layer suitable to be fixed on a sensor surface allows the replacement of the main biological components by non-biologically chemically inert and stable synthetic compounds without impairing the function of the sensor in measuring the blood coagulation time.

Preferably said non-biological chemically inert and stable synthetic compounds are formed by the resin itself, which can be designed to be negatively charged on its own by introducing suitable negatively charged sidechains into its polymer backbone.

In another embodiment of the invention the negatively charged surface layer is provided by non-biological chemically inert and stable compounds, which are integrated into the lacquer system. Negatively charged materials can be co-immobilized within the resin together with the above mentioned thrombin substrate. These said non-biological chemically inert and stable compounds are selected from the group consisting of negatively charged clay materials such as bentonite, Kieselgur or other negatively charged additives such as oxidized carbon nanotubes.

In a preferred embodiment of the invention said lacquer system comprises a non-conducting anodic electrodeposition lacquer, preferably said lacquer is selected from the group consisting of negatively charged acrylic acid based electrodeposition polymers with variable monomer composition. The said lacquer system is becoming less soluble upon changing the pH value. Lacquer systems which become less soluble upon increasing the pH value are called cathodic electrodeposition polymers while lacquer systems which become less soluble upon decreasing the pH value are called anodic electrodeposition polymers.

In one embodiment of present invention the at least one substance with a detection function comprises a specific reagent including at least one chemical substrate with one terminal link which can be cut off by an enzyme to give a free-diffusing redox active compound, preferably wherein said chemical substrate reagent with one terminal link which can be cut off by an enzyme to give a redox active compound is an amperogenic thrombin substrate and the enzyme is thrombin.

The present invention further encompasses a method of producing such blood coagulation measuring sensor.

In a preferred embodiment the method comprises depositing the resin layer comprising a lacquer system on a surface and fixing the resin layer on the surface by drying.

### Brief description of the Drawings

Fig. 1: Perspective view of a measuring apparatus for measuring the blood coagulation time in a sample using the sensor of present invention.
Fig. 2: Schematic diagram as already shown in US6352630 depicting the chemical reaction, which allows a current to be generated across the electrodes of the sensor.
Figs. 3A-D: Comparison of immobilization methods of the deposition solution on the sensor surface. Electrodeposition versus drying in absence of Thromborel. A) Polymer precipitation with 10 deposition pulses; B) Polymer precipitation by drying; C) results of measurement for electrodeposition; C) results of measurement for drying.
Figs. 4A and 4B: Comparison of one month storage stability of sensors where the deposition solution was immobilized on the sensor surface by electro-deposition and by simple drying.
Figs. 5A and 5B: Sensors fabricated with addition of small amounts of carbon nanotubes.
Figs. 6A and 6B: Sensors fabricated in presence of an increased amount of negatively charged carbon nanotubes.

### Detailed description of the Invention

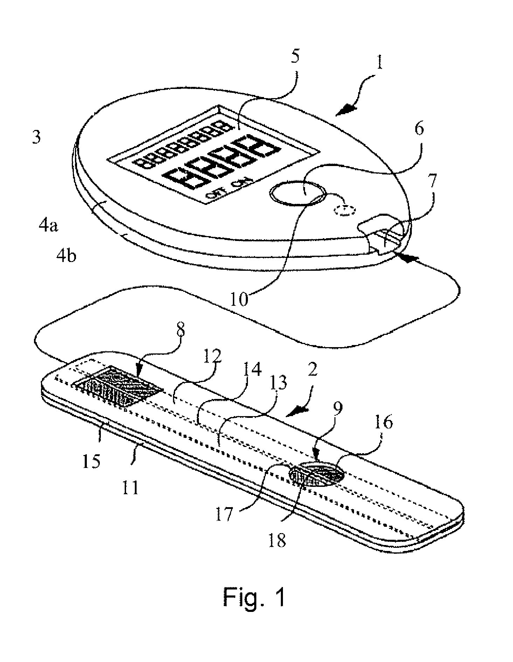

Present invention relates to a blood coagulation time measuring sensor. The measuring apparatus shown in Fig.1 shows one example of a setup in which a blood coagulation time measuring sensor according to present invention can be used for measuring the blood coagulation time in a sample. This measuring apparatus is known form US 6,352,630.

Measuring apparatus 1 includes a case 3 constructed by assembling two moulded plastic elements 4a, and 4b, of which the top element 4a includes a window for a display panel 5, and an opening for a control button 6 allowing access to the various display modes.

The electronic circuit included in the case is an adaptation of the circuits used for glucose analysis, for example by amperometry as indicated previously, and differs only therefrom in the different electric signal parameters in order to display a coagulation time.

Measuring apparatus 1 also includes a slit 7, arranged substantially between the two portions 4a, 4b of case 3, into which a portion of the sensor 2 including a contact zone 8 is introduced. The other portion of sensor 2 including a measuring zone 9 intended to receive a blood sample, will remain outside apparatus 1 during the entire measuring time. In most of the measuring apparatuses of the prior art, the sample is introduced inside the apparatus into an enclosure generally thermoregulated at 37°C, which leads to additional power consumption.

In the system according to the invention, in which the sample is not introduced into a thermoregulated enclosure, it is easy to provide, if necessary a heat probe 10 on the apparatus in proximity to slit 7 to adjust the electric signal parameters as a function of the ambient temperature, with a much lower power consumption than that necessary to thermoregulate an enclosure.

The sensor 2 includes a thin plastic support 11, made for example of PET, supporting over its entire length two current collectors 12, 13 separated by a small space 14 which insulates them electrically.

Support 11 and collectors 12, 13 are coated with an insulating coating 15 into which two windows 8, 9 are cut, close to each end, allowing portions of collectors 12, 13 to appear. A first window 8 allows sensor 2 to be electrically connected to measuring apparatus 1, while second window 9 constitutes the measuring zone, the visible collector portions respectively forming the working electrode 16 and the reference electrode 17.

Working electrode 16 is for example made by laminating a thin strip of platinum and reference electrode 17 by laminating a thin strip of subsequently chlorinated silver to form at the same time a reference electrode. It is also possible to provide separately, in measuring zone 9, a working electrode, a reference electrode and a counter electrode. The working electrode is coated with a specific reagent 18 described in more detail hereinafter to allow the prothrombin time (PT) to be measured.

The reagent composition of present invention that is deposited on the sensor includes a chemical substrate of which a terminal fraction can be selectively cut off by the thrombin enzyme by releasing a charged and/or redox active group.

Suitable substrates and groups of substrates have been described previously in US 6,352,630 and in particular in US 6495336.

As described in US6352630, the suitable oligopeptidic substrates have a stereospecific configuration of a site of the thrombin enzyme to allowing cutting off of a charged terminal portion generally designated to be the leaving group (LG). The substrate used is characterised in that the oligopeptidic derivative or one of its salts includes in addition to arginine at least one other radical amino-acid selected from among 2-aminobutyric acid (Abu), alanine (Ala), 3-cychlohexylalanine (Cha), 2-cyclohexylglycine (Chg), phenylalanine (Phe), pipecolic acid (Pip), proline (Pro), valine (Val) and glycine (Gly) said amino-acids being able to be in the L, D or DL form. Likewise the leaving group (LG) which, in the conditions of the invention, must both react easily with the acid function of arginine (Arg), be able to be cut off by thrombin and have a sufficient charge, is preferably selected from among aniline, quinolylamine and naphtylamine derivatives possibly substituted by one or more substituents selected from among an halogen, an hydroxy, amino, nitro, alkyl, alkoxy, alkanoyl, anilino and aminophenyl radical, which may be substituted.

The oligopeptide is immobilised on the working electrode via the opposite end of its chain to that including the leaving group LG. The first amino-acid of the oligopeptic chain, but preferably a hydrogen of its terminal amino function is therefore replaced by a protective group R, selected from among the Boc (terbutoxycarbonyl), Tos (paratoluenesulfonic), t-Bups (ter-butylphenylsulfonyl), Mes (methylsulfonyl), Naps (2-naphtylsulfonyl), Bzo (benzoyl), Z (benzyloxycarbonyl), isopropylsulfonyl, camphosulfonyl acids.

According to a preferred embodiment, the protective group, the α-amino-acids and their chain formation, as well as the leaving group are selected so as to have the following oligopeptidic substrates :
Z-Gly-Pro-Arg-3-chloro-4-hydroxyanilide
Tos-Gly-Pro-Arg-3-chloro-4-hydroxyanilide
Boc-(D)-Chg-Gly-Arg-3-chloro-4-hydroxyanilide
H-(D)-Chg-Gly-Arg-3-chloro-4-hydroxyanilide
Z-Gly-Pro-Arg-2- chloro-4-hydroxyanilide
   and their compatible mineral or organic salts.

The oligopeptides in the composition of the specific reagent are generally used in the form of one of their salts, formed for example with hydrochloric acid (HCl), acetic acid or trifluoroacetic acid (TFA).

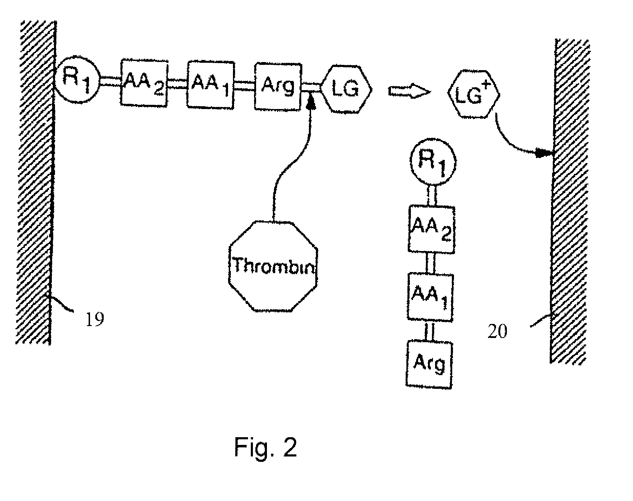

FIG. 2 is a schematic diagram as already shown in US6352630 that depicts the chemical reaction which allows a current to be generated across electrodes 20 and 21, which are connected by an electronic detection circuit (not shown). The substrate, which can be schematically represented by the formula R1-AA2-AA1-Arg-LG in which AA1 and AA2 represent other amino-acids as previously indicated, is connected to working electrode 20 by the group R1 which orients the oligopeptide, and the other end of whose chain includes a leaving group LG as defined previously. In the left part of the diagram, it is seen that the thrombin enzyme selectively cuts off the connection between the arginine and said leaving group LG. In the right part of the diagram, it is seen that the leaving group released can migrate towards electrode 21 and generate a current which will be proportional to the number of leaving groups LG released, and thus to the quantity of thrombin formed in the sample.

Further suitable thrombin substrates for use in the sensor of present invention as already described in US 6495336 are:
Oligopeptide derivatives of the formula (I)

   R¹——(D,L),D or

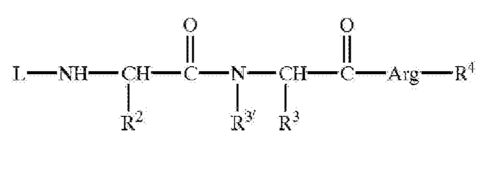

   in which
   R¹ is
   (a) a hydrogen atom, a C₂₋₈-alkanoyl group optionally having an amino group in the ω position, a phenyl-C₂₋₄-alkanoyl group whose phenyl radical is optionally substituted in the p position by an amino group; or
   (b) a cyclohexylcarbonyl group which is optionally substituted in the 4 position by an aminomethyl radical, a benzoyl group which is optionally substituted in the o or p position by methyl, amino or halogen, a C₁₋₈-alkoxycarbonyl group, a benzyloxycarbonyl group which is optionally substituted in the p position by methoxy, methyl or chlorine; or
   (c) a radical of the formula -SO₂-R⁵ where R⁵ can be a C₁₋₆-alkyl radical, an optionally substituted aryl or heteroaryl radical or a radical of a bicyclic terpene derivative; or
   (d) a group of the formula -CO-CH(R⁶)-NH-R⁷, where R⁶ is hydrogen, a C₁₋₆-alkyl radical, a 1- or 2-hydroxyethyl radical, a methylmercaptoethyl radical, an aminobutyl radical, a guanidinopropyl radical, a carboxy-C₁₋₄-alkyl radical, a carboxamido-C₁₋₄-alkyl radical, a phenyl-C₁₋₄-alkyl radical whose phenyl radical is optionally substituted by OH, halogen, C₁₋₄-alkyl or methoxy, or a cyclohexyl or cyclohexylmethyl radical whose ring is optionally substituted by OH, halogen, C₁₋₄-alkyl or methoxy, or a nitrogen-containing heteroaryl- C₁₋₄-alkyl radical with 3 to 8 carbon atoms in the heterocyclic system, where the group -CO-CH(R⁶)-NH-R⁷ may be racemic or have the D or L configuration, and R⁷ can be a group of type (a), (b) or (c); or
   (e) a group of the formula 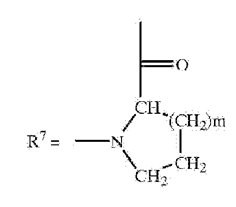
   where R⁷ has the above meaning, m can be 1 or 2, and one of the methylene groups can be substituted by hydroxyl, carboxyl, C₁₋₄-alkyl or aryl- C₁₋₄-alkyl;
   R² is hydrogen, C₁₋₆-alkyl, C₁₋₂-hydroxyalkyl, C₁₋₄-alkoxy-C₁₋₆-alkyl, benzyloxy-C₁₋₂-alkyl, an ω-carboxy-C₁₋₃-alkyl radical, an ω-C₁₋₄-alkoxycarbonyl-C₁₋₃-alkyl radical, an ω-benzyloxycarbonyl-C₁₋₃-alkyl radical or a cyclohexyl, cyclohexylmethyl, 4-hydroxycyclohexylmethyl, phenyl, benzyl, 4-hyroxybenzyl or imidazolyl-4-methyl radical;
   R³ is s
   (a) hydrogen or C₁₋₄-alkyl and R ^{3'} is hydrogen; or
   (b) together with R^{3'} a tri- or tetramethylene group, where one of the methylene groups can be substituted by hydroxyl, carboxyl, C₁₋₄-alkyl or aryl-C₁₋₄-alkyl; and
   _{R}⁴ i s
   (a) an aniline residue of the formula 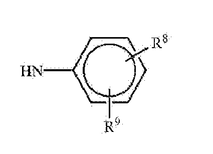 where R⁸ can be hydroxyl or amino and R⁹ can be hydrogen, halogen, amino, nitro, C₁₋₄-alkyl, C₁₋₄-alkoxy or C₁₋₄-alkanoyl; or
   (b) a quinoline residue of the formula 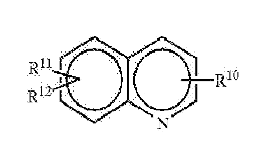
   where one of R¹⁰, R¹¹ and R¹² is an -NH group via which the quinoline residue is linked to the Arg residue, a second one can be hydroxyl or amino, and the third one can be hydrogen, hydroxyl or amino; and the salts thereof.

These oligopeptide derivatives are cleaved by enzymes of the peptide hydrolase enzyme class (E.C. 3.4.), in particular proteinases (E.C. 3.4.21-99) and inhibitors thereof, of the blood coagulation system, of the fibrinolytic system and of complement, in particular of thrombin. They thus serve as substrates for the quantitative and qualitative determination of the abovementioned enzymes, in particular thrombin, in complex sample liquids, in particular capillary blood.

As detailed in US6352630 a biological factor, such as thromboplastin is needed to allow the thrombincascade to proceed so that the prothrombin time can be measured.

In contrast to that the present invention is based on the principle that the main biological components of the blood coagulation cascade are replaced by non-biologically chemically inert and stable compounds. The specific reagent mix of the present invention does therefore not contain thromboplastin, but instead a lacquer material that allows on the one hand immobilization of an amperogenic thrombin substrate as described above and at the same time provides for a negatively charged surface as mentioned as a requirement for the blood-coagulation cascade to proceed. Present invention shows that surprisingly no additional biological factors are required in such reagent mix to initiate blood coagulation.

Preferably, said non-biological chemically inert and stable synthetic compounds are formed by the resin itself, which can be designed to be negatively charged on its own by introducing suitable negatively charged sidechains into its polymer backbone.

Lacquer materials that provide for such advantages and are suitable for use in the sensor of present invention are described in US2003/0153061. The reagent mix of present invention comprises anodic electrodeposition lacquers that are deposited onto the substrate surface.

Different methods can be used for depositing the reagent mix of present invention onto the substrate surface. The sensor can for example be prepared by manual dispensing using a micropipette, by automatic dispensing using a micropump and a positionable syringe, by electrochemically induced deposition of the electrodeposition lacquer in the presence of the thrombin substrate and additives, by drop coating of the deposition solution followed by drying, or piezospotting.

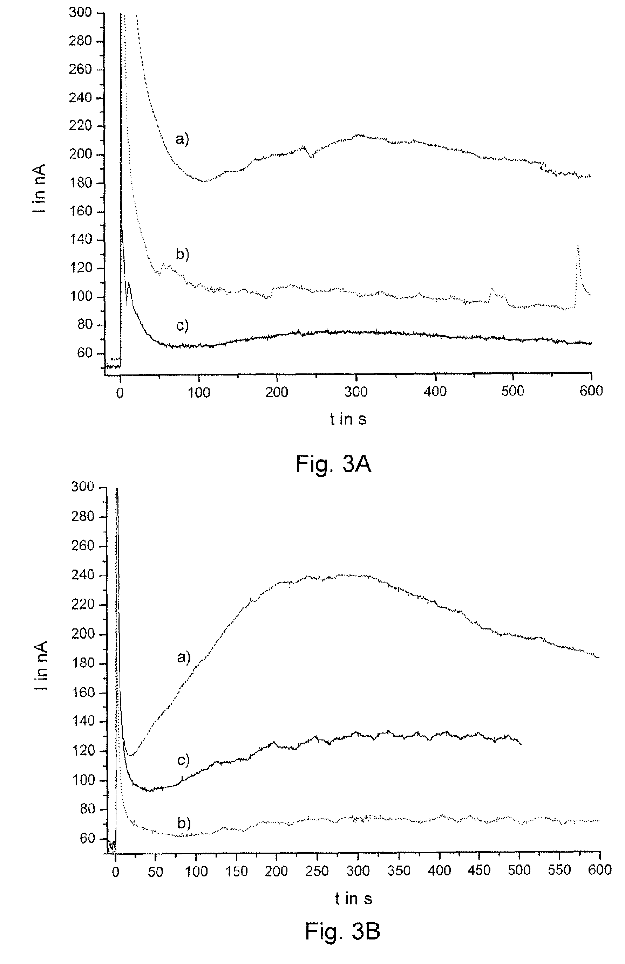

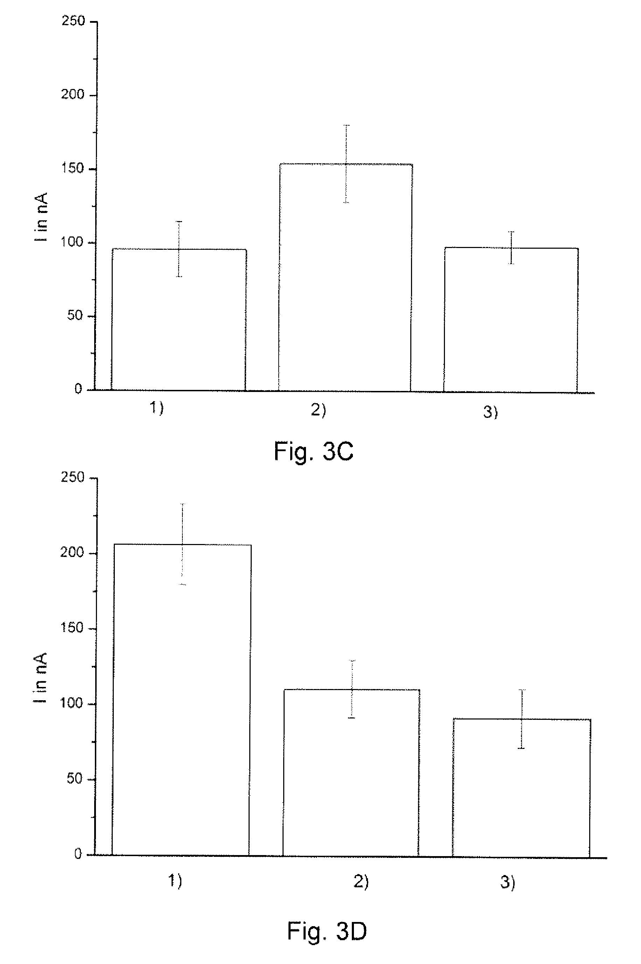

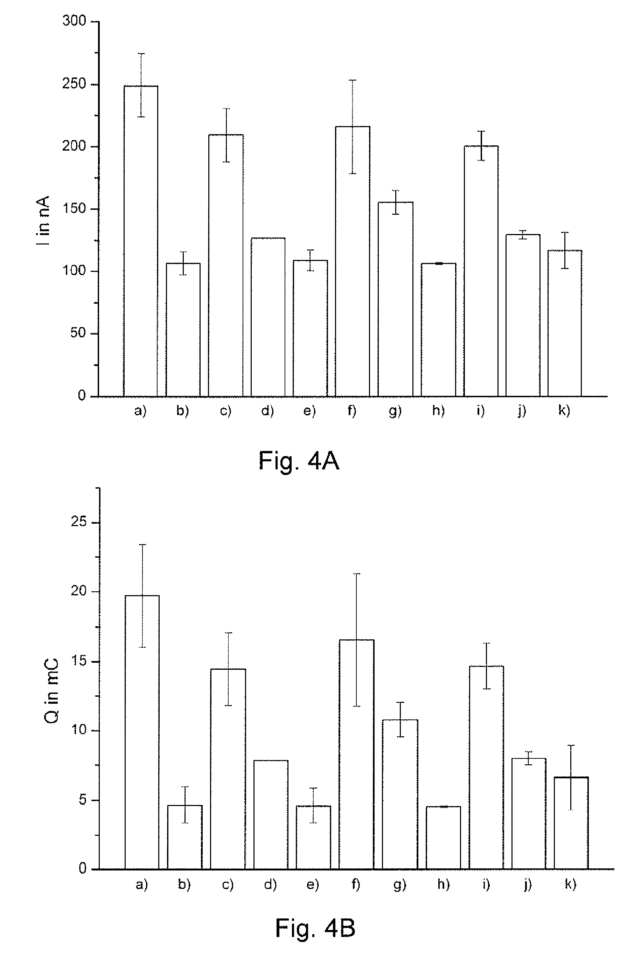

In a preferred embodiment of present invention the method comprises depositing the resin layer comprinsing a laqcuer system on a surface and fixing the resin layer on the surface by drying. Experiments have shown that such simple method is suitable for producing highly stable sensors as shown in example 3 and figures 3 and 4. The major advantage is the simplicity of this yet highly effective method.

The other method as used in present examples is the method of electro-deposition lacquering, which is used industrially for protection against corrosion in automobile chassis and components, radiators and drink cans. In anodic electro-deposition the protons released by decomposition of water at the anode are used to neutralise and thus precipitate a soluble polymer, negatively charged on account of carboxylate groups. The film is then cross-linked on the surface to form an impermeable coating, which has great mechanical and chemical resistance.

For electrodeposition the reagent mix of present invention contains a resin suitable for electrodeposition. On account of the creation of protons at the surface of the target electrode, resin deposits are created, which incorporate and thus immobilise any molecules or compounds simultaneously present in the reagent mix.

Arbitrary known resin emulsions that are suitable for anodic electro-deposition can be used as the resin emulsion. Among these are organic compounds of higher molecular weight in the range from 300 to 50000; 600 to 30000; 1000 to 20000; 800 to 10000; 1000 to 15000, also however higher molecular weights, insofar as they can be dissolved or emulsified, from the previously given values up to 50000, 70000, 100000, 250000 or even 1000000, especially resins with electric charges of an ionic nature, which are created by functional groups on the constituent monomers. Examples are modified polystyrols, olefins, polyamides and vinyl and acryl compounds, also however biochemically interesting polymers, such as proteins, polysaccharides with functional groups, vegetable gums, oligo and polynucleotides. α , β olefinic unsaturated carboxylic acid polymers have proved themselves in particular for the anodic deposition.

A preferred resin for the resin emulsion used for anodic electro-deposition is a resin preparation on the basis of, a copolymerisate containing carboxylic groups, masked isocyanate groups, hydroxyl and ether groups, which can be dissolved or dispersed in water by at least partial salt formation with ammonia or an organic base, wherein the copolymerisate contains polymerised:
1. At least one α, β olefinic unsaturated carboxylic acid with 3 to 5 carbon atoms or a half-ester of an α,β olefinic unsaturated dicarboxylic acid containing 3 to 5 carbon atoms ;
2. 10 to 35 percent by weight of an N(1-alkenyl) isocyanate masked with CH-, OH- or NH-active masking means ;
3. 20 to 50 percent by weight of an adduct of an epoxide resin on the basis of bisphenol A and epichlorhydrin with a molecular weight between 380 and 3500 and an olefinic, unsaturated alcohol containing 3 to 5 carbon atoms ;
4. 5 to 64 percent by weight of one or more olefinic, unsaturated compounds not recited under 1. to 3. which can be copolymerised, with the provision that the copolymerisate has a mean molecular weight between 1000 and 20000, includes the component (1) polymerised in such an amount that the acid number of the copolymerisate amounts to 35 to 150 mg KOH/g and the sum of the percentages recited under 1 to 4 is 100.

The copolymerisate mentioned above has a mean molecular weight between 1000 and 20000. The components are polymerised in such an amount that the acid number of the copolymerisate amounts to 35 to 150 mg KOH/g. The sum of the percentages recited under 1 to 4 amounts to 100. It is to be emphasised that the equivalent ratio of the reactive hydrogen atoms of the component (3) to the masked isocyanate groups in the copolymerisate preferably amounts to about 1:1. Furthermore an adduct of vinylisocyanate or propenylisocyanate and cyclohexanol, terbutanol, triazabenzol or [epsilon]-caprolactam is preferably used with the molecular ratio 1:1 1 of isocyanate/masking means. In particular the copolymerisate contains as component (1) acrylic or methacrylic acid, as component (2) vinylisocyanate masked with [epsilon]-caprolactam, as component (3) a conversion product of an epoxide resin of bisphenol A and epichlorhydrin with a mean molecular weight of about 900 and allyl alcohol, as well as polymerised 2-ethylhexylacrylate or butylacrylate as component (4).

Also preferred is a resin-like mass which is used as a mixture of a water soluble resin-like material and a water insoluble resin-like mass. The mixture is dispersed in an aqueous medium, in which water is the main constituent. The water soluble resin-like materials are polymers and are rendered water soluble through incorporation of sufficient hydrophilic groups into the polymer. The hydrophilic groups can be ionic salt groups, for example anionic salt groups, such as carboxylic acid and sulfonic acid salt groups, or cationic salt groups, such as amine salt groups and quaternary ammonium salt groups. The preferred hydrophilic groups are anionic groups and especially preferred are salts of carboxylic acid groups. A polymer is usually produced with carboxylic acid groups and then neutralised with a water soluble basic compound, such as an organic amine or an alkali metal hydroxide.

The concept "water-soluble" means in this connection that the resin-like materials, be made soluble in water, can be dispersed with a resin solids component of up to 25%, usually 1 to 20 percent by weight, without the aid of externally added tensids. The solution or dispersion frequently appears optically transparent or translucent, where the resin is present in the dispersed phase and has a mean particle size of 0.12 µm and less, usually less than 0.03 µm. The mean particle size of the water-soluble resin-like materials can be determined by a light scattering method.

The preferred lower molecular, water-soluble polymers are acrylic copolymers which have an anionic charge, preferably a carboxylic acid salt group and especially preferred a carboxylic acid group neutralised by an organic amine.

Among the lower molecular acrylic copolymers are polymers, produced by copolymerisation of an α,β ethylenic, unsaturated carboxylic acid with a methacrylic acid ester and/or acrylic acid ester, and in general acrylic polymers are suitable which comprise as the main component a methacrylate ester of a C₁-C₈ alcohol and a small proportion of an acrylate ester with a C₁-C₈ alcohol. The following compounds are common methacrylate esters and acrylate esters: ethyl acrylate, propylacrylate, isopropylacrylate, butylacrylate, isobutylacrylate, secondary butylacrylate, hexylacrylate, 2-ethylhexylacrylate, octylacrylate, methylmethacrylate, propylmethacrylate, isobutylmethacrylate, butylmethacrylate, secondary butylmethacrylate and tertiary butylmethacrylate.

The acryl polymers which are employed contain 0.1 to 20 percent by weight of a polymerised α,β ethylenic, unsaturated carboxylic acid unit. α,β_ethylenic unsaturated carboxylic acid monomers which can be used are methyacrylic acid, acrylic acid, itaconic acid, ethacrylic acid, propylacrylic acid, isopropylacrylic acid and homologues of these acids. Methacrylic acid and acrylic acid are preferred. The percentage of acid is so set that the required acid number is created in the acrylic polymer. The acid number of the acrylic polymer should usually be adjusted in that way that it amounts to about 30 to 100 % of the resin solids component. The numerical average molecular weights of the water-soluble acrylic polymers preferably lie in the range from 10 000 to 30 000.

The acrylic polymers can also contain hydroxyl side groups, which are obtained by copolymerisation of hydroxyalkylacrylates or hydroxyalkylme-thylacrylates with the abovementioned acrylic esters. The hydroxyl groups-side groups provide places for subsequent curing, such as with an aminoplast or a masked isocyanate. 5 to 15 percent by weight of the utilised acrylic polymer are preferably from a hydroxyalkylacrylate or methacrylate ester. In general usable hydroxyalkylacrylates and methacrylates contain 1 to 8 carbon atoms in the alkyl group and are for example hydroxyethylacrylate, hydroxypropylacrylate, hydroxybutylacrylate, hydroxyethylmethacrylate, hydroxypropylmethacrylate, hydroxybutylmethacrylate, hydroxyhexylmethacrylate and hydroxyoctylmethacrylate for example.

Further vinyl copolymerisable compounds can be used, in order to form a part of the usable acrylic polymers, such as styrol, vinyltoluol, acrylamide, vinylxylol, allyl alcohol and acrylnitrile.

In an especially suitable acrylic polymer the polymer consist essentially of a hard component, namely either styrol or a lower alkylmethacrylate, wherein the acryl group contains 1 to 2 carbon atoms, or a mixture of styrol and lower alkylmethacrylate, such as ethylacrylate, a soft component, namely a lower alkylmethacrylate with 3 to 8 carbon atoms in the alkyl group or lower alkylacrylate with 2 to 8 carbon atoms in the alkyl group, a hydroxy lower alkylmethacrylate or acrylate with 1 to 4 carbon atoms in the alkyl group and an [alpha], β-ethylenic, unsaturated carboxylic acid, as described above.

As well as water-soluble acrylic resins, polyesters which are produced from saturated or aromatic polycarboxylic acids and a polyol are suitable for the polymers having a lower molecular weight. Typical saturated aliphatic dicarboxylic acids are anhydrides with 2 to 10 carbon atoms, such as butandioic acid, azelainic acid and adipinic acid, which are suitable for the production of these polyesters. Examples of aromatic dibasic acids or their anhydrides are phthalic acid and trimellitic acid. The acid amount for the polyester is so set that the desired acid number is achieved, which should be 20 to 85 for the polyester. Many polyols can be converted with the above cited acids to produce the desired esters. Especially suitable diols are for example ethyleneglycol, 1, 4-butanediol, neopentylglycol, sorbitol, pentaerithritol and trimethylolpropane.

Alkyd resins, such as polymer esters, produced by condensation of polyhydric alcohol, such as glycerineethyleneglycol, and a drying fatty acid, such as linseed oil and tallol are also suitable as water-soluble polymers. A further component, which is usually added in order to achieve the desired acid number is for example α,β-ethylenic unsaturated dicarboxylic acid or the anhydride of the acid, such as maleic acid or maleic acid anhydride.

These alkyd resins should preferably have a numerical average molecular weight from 1 000 to 2 500 and an acid number from 20 to 85.

A further lower molecular carboxylic acid polymer which can be used for producing electro-deposition is a polymer of styrol and an ethylenic, unsaturated alcohol having 3 to 10 carbon atoms, such as allyl alcohol. The polymer can furthermore be converted with drying fatty acids and with an acid component such as those which have been recited above, in order to achieve the required acid number, usually in the range from 20 to 80. The numerical average molecular weights of the styrol-allyll-alcohol polymers usually lie in the range from 1 000 to 10 000.

Epoxide esters are also suitable as water-soluble resins of lower molecular weight. These materials are obtained by partial esterification of an epoxide resin with a customary drying fatty acid, such as those which have been recited above, and this resin is then esterified with an [alpha], β-etheylenic, unsaturated dicarboxylic acid or an anhydride thereof, such as those which have been mentioned above. The epoxide resin itself is preferably a polyglycidylether of a bisphenol, such as bisphenol A.

Examples of further suitable resin-like polymers of lower molecular weight are the neutralisation products of unsaturated carboxylic acids, such as maleic acid or anhydride and a drying oil, such as linseed oil.

A further composition for the anodic electro-deposition comprises (A) 15 to 60 percent by weight water, (B) 15 to 60 percent by weight of one or more organic solvents, (C) 0.1 to 20 percent by weight of a copolymer of (a) 10 to 75 percent by weight of one or more copolymerisable [alpha], β -olefinic, unsaturated compounds which are immiscible or partly miscible with water and (b) 25 to 90 percent by weight of one or more water-soluble copolymerisable N-vinyl compounds and (D) 10 to 79 percent by weight of one or more finely divided pigments or fillers or mixtures of pigments and fillers, dispersed in the mixture (A), (B), (C), wherein the sum of the percentages of (A), (B), (C) and (D) is 100. In particular component (C) in this is a copolymer which can be produced by solvent polymerisation for example. It is used in an amount of 0.1 to 20 percent by weight, preferably 3 to 8 percent by weight, and contains as copolymerisable units (a) 10 to 75 percent by weight, preferably 20 to 40 percent by weight, of one or more copolymerisable αβ-ethylenic, unsaturated compounds which are immiscible with water or only partly miscible, and (b) 25 to 90 percent by weight, preferably 60 to 80 percent by weight of one or more water-soluble copolymerisable N-vinyl compounds.

The sum of the percentages of (a) and (b) is 100. Preferred αβ-ethylenic, unsaturated compounds (a) which are immiscible with water or only partly miscible are vinyl esters of C₂-C₁₈-monocarboxylic acids, for example vinylacetate, vinylproprionate, vinylpivalate, vinyl-2-ethylhexanoate and vinylstearate and/or acrylic acid esters or methacrylic acid esters of C₄-C₁₈-alcohols, for example butylacrylate and butylmethacrylate, 2-ethylhexylacrylate, octylacrylate and octadecylacrylate. A particularly preferred monomer is vinylproprionate. Also suitable are derivatives of acrylamide or methacrylamide, vinylether and/or vinyl aromatics such as styrol, which are insoluble or only a little soluble in water.

Examples of preferred water-soluble N-vinyl compounds (b) are N-vinylpyrrolidone, N-vinylpiperidone and N-vinylimidazole.

Preferred electro-deposition emulsions according to present invention are selected from Resydrol lacquers, Resydrol AY 498w/35WA, Resydrol AM 410w/67WABG, Cathodip GY83-0270 0005, WA-Tauchlack von FreiLack GmbH, Freitherm-Elektrotauchlack KTL automotive "upgrade", BASF GY80-0636-0001 KM 1.

Water can optionally be added to the electro-deposition lacquer suspension available in the trade, where the water should be as pure as possible. Water of HPLC quality is preferred. Furthermore any other materials which are usually added to resin layers can be used. Such materials are for example softeners, emulsifying agents, adhesion promoters, hydrophobing or hydrophilic materials, cross-linking agents, anti-foaming agents or the like.

Suitable additives which mainly exert the function of an emulsifier or cross-linking agent in the production and preparation of the electro-deposition lacquer suspension are anionic, cationic or amphophilic tensids, above all non-ionogen tensids. In this connection suitable commercially available products are set out below:
1. Triton (X-100, X-114, X-405 etc.): alkylphenylpolyethyleneglycol (Fluka)
2. Tween (20, 40, 60 etc.):
   Tween 20: polyoxyethylenesorbitanemonolaurate (Merck)
   Tween 40: polyoxyethylenesorbitanemonopalmitate (Merck)
   Tween 60: polyoxyethylenesorbitanemonostearate (Merck)
   Tween 65: polyoxyethylenesorbitanetristearate (Merck)
   Tween 80: polyoxyethylenesorbitanemonooleate (Merck)
   Tween 85: polyoxyethylenesorbitanetrioleate (Merck)
3. Nonidet P40: octyl-phenyl-polyethyleneglycol (Fluka)
4. Brij (35, 56, 58 etc.): (Merck)
   Brij 35: polyoxyethylenelaurylether
   Brij 56: polyoxyethylene-(10)-cetylether
   Brij 58: polyoxyethylene-(20)-cetylether
5. octyl [beta]-glucoside (Pierce)
6. octyl [beta]-thioglucopyranoside (Pierce)
7. SDS: sodiumlauryl-sulfate (Fluka)
8. CHAPS: 3-[(3-cholamidopropyl)-dimethylammonio]-propanesulfonate (Fluka)
9. CHAPSO: 3-[(3-cholamidopropyl)-dimethylammonio]-2-hydroxy-propanesulfonate (Fluka)

### Examples

Example 1: Preparation of the resin layer for deposition on the sensor and immobilization of the sensing components on the sensor surface.

The following solutions (#1 to #5) were prepared for formation of the sensors:
#1. Hepes-buffer (50 mM, pH7.4, KCI 100 mM): 1.19g HEPES and 0.75g KCI in 90 ml DI water. pH value was adjusted to 7.4 with NaOH and increasing the volume with DI water to 100 ml
#2. Na-deoxycholat solution: 3.8 mg Na-deoxycholat in 1500 µl Hepes buffer (#1)
#3. 10 µl phosphatidylserin + 35 µl Na-deoxycholat solution (#2)
#4. Activation solution: 45 µl solution #3 + 1080 µl Hepes buffer (#1)
#5. Deposition solution: 0.3 mg thrombin substrate + 890 µl activation solution #4 (5 min in ultrasonic bath) + 30 mg spectral coal, 4 mg bentonite SF and 110 µl resydrol AY

The deposition solution can be varied in order to co-immobilize additional compounds on the sensor surface. In that case, the composition of solution #5 was kept constant with respect of the amount of thrombin substrate, the volume of the activation solution #4 and the amount of resydrol AY. The exact composition of the deposition solution is given for each example if different from solution #5.

### Example 2: Immobilization of the sensing components on the sensor surface by means of electrodeposition.

The sensors were washed in isopropanol for 15 min in an ultrasonic bath, rinsed with water and dried. A droplet of 50 µl of the deposition solution (#5) was pipetted on the sensor surface, which was fixed horizontally in a holder. A Ag/AgCl/3 M KCI reference electrode and a Pt wire counter electrode were inserted into the droplet. For electrodeposition protons have to be locally produced at the working electrode surface, which is initiated by potential pulses to a potential of at least 2.2 V, invoking water oxidation. The number of pulses with a duration of at most 2 s, preferentially 0.5 s to the deposition potential followed by a resting phase of at least 2 s, preferentially 5 s at a no effect potential below 1 V preferentially 0 V can be varied and is determining the thickness of the deposited laquer film. The number of potential pulses is at least 1, better 5 and preferentially between 10 and 50 pulses. After the electrodeposition the sensor is rinsed with buffer solution (#1).

### Example 3: Immobilization of the sensing components on the sensor surface by means of dispensing and drying.

The sensors were washed in isopropanol for 15 min in an ultrasonic bath, rinsed with water and dried. 5 µl of the deposition solution (solution #5) were deposited on the working electrode of the sensor manually using a pipette or automatically using a syringe pump-based dispenser. After placing the deposition solution on the surface of the working electrode the modified sensor was dried for 3 hours at 37°C.

### Example 4: Amperometric determination of the blood coagulation time.

For the determination of the blood coagulation time a sensor was contacted to a potentiostat, preferentially a meter as shown in Figure 1. Using a pipette, either 7µl control plasma N (N), control plasma P (P) or blood (B) were dropped on the dry sensor surface. In the moment of the sample addition, the electrical circuit is closed and a working potential of +0.3 V is applied to the working electrode while the current is simultaneously detected. The time of addition of the sample was set to be the zero point of the measurement.The current was recorded over time and the course of blood coagulation can be monitored via the oxidation current of the thrombin substrate. During blood coagulation the thrombin substrate is cleaved and the thus mobile redox active tag can reach the electrode surface to be oxidized at the applied potential. The current represents the number of cleaved thrombin substrate molecules in time and hence are correlated with the coagulation.

### Example 5: Comparison of immobilization methods.

It was tested whether it is possible to omit the electrodeposition step (as described in example 2) and instead only dry the deposition solution on the sensor surface (as decribed in example 4) in order to simplify the production of the sensors. For measurements the setup as described in figure 1 was used. The applied potential pulse profile was 2.2 V for 0.2 sec and 0 V for 5 sec and was repeated ten times. The potential was applied to the working electrode of the sensor and the reference and counter electrode, which was immerged into the substrate solution. In figure 3 the results of the measurements with sensors that were produced by either fixing the deposition solution with an electrodeposition step or by simple drying are shown.

Figure 3 shows current response curves over time for sensors prepared by A) polymer precipitation with 10 deposition pulses from solution #5 (in absence of the biological activator Thromborel S in the deposition solution); dashed line (a): plasma N; pointed line (b): whole blood; black line (c): plasma P; B) polymer precipitation by drying without Thromborel S in the deposition solution; dashed line (a): plasma N; pointed line (b): whole blood; black line (c): plasma P; C) bar diagram summarizing the results for the electrodeposited sensors (current after 200 s; 1. blood (n=2); 2) plasma N (n=9); plasma P (n=7); D) bar diagram summarizing the results for the dried sensors 1) plasma N (n=17); 2) blood (n=2); 3) plasma P (n=14).

Example 6: Comparison of long term stability of sensors produced by electrodeposition (see example 2) and by drying (see example 3).

The sensors were placed in a plastic tube as usually used to distribute such sensors (cylinders with 2 cm diameter closed with a srew cap). These containers were placed on a wip-board, which was wipping with a frequency of 0.1 Hz and allowed to roll from right to left and back for up to 33 days. Another set of similarily-fabricated sensors were placed in an incubator at 37° for up to 33 days. Sensors incubated or shaken for 0, 3 and 33 days were used for measurements as described in example 4 using the setup as shown in figure 1. Figure 4 shows the current over time measured with sensors after different incubation times as well as the current and the transfered charge measured after 200 and 350 s.
A) current after 200 s
   a) directly after sensor preparation, plasma N (n=6); b) directly after sensor preparation, plasma P (n=6); c) after 3 days, plasma N (n=8); d) after 3 days, blood (n=1); e) after 3 days, plasma P (n=6); f) after 33 days, plasma N (n=5); g) after 33 days, blood (n=4); h) after 33 days, plasma P (n=2); i) after 33 days continuous shaking, plasma N (n=4); j) after 33 days continuous shaking, blood (n=2); k) after 33 days continuous shaking, plasma P (n=2);
      ■ Deposition solution #5a: 0.3 mg thrombin substrate + 890 µl activation solution B (15 min in ultrasonic bath) + 29.9 mg spectral coal, 4 mg Kieselguhr and 110 µl resydrol AY
B) Charge after 200 s and 300 s
   a) directly after sensor preparation, plasma N (n=6); b) directly after sensor preparation, plasma P (n=6); c) after 3 days, plasma N (n=8); d) after 3 days, blood (n=1); e) after 3 days, plasma P (n=6); f) after 33 days, plasma N (n=5); g) after 33 days, blood (n=4); h) after 33 days, plasma P (n=2); i) after 33 days continuous shaking, plasma N (n=4); j) after 33 days continuous shaking, blood (n=2); k) after 33 days continuous shaking, plasma P (n=2);
      ■ Deposition solution #5a: 0.3 mg thrombin substrate + 890 µl activation solution B (15 min in ultrasonic bath) + 29.9 mg spectral coal, 4 mg Kieselguhr and 110 µl resydrol AY

### Example 7: Sensors fabricated in presence of negatively charged carbon nanotubes.

Sensors were fabricated following example 3 (dispensing and drying) using additionally a solution of negatively charged carbon nanotubes (solution #6), which were added to the deposition solution forming the solution #5c:
■ Solution #6: 15.7 mg carbon nanotubes were stirred in 100 ml HNO₃ at 110°C für 90 min; the solution was filtrated, the nanotubes were washed with DI water and dried at 80°C for 3 h
■ Deposition solution #5c: 0.4 mg thrombin substrate + 1000 µl activation solution #4 (15 min in ultrasonic bath). To 445 µl of this solution 1 mg negatively charged nanotubes and 55 mg resydrol AY was added. The solution was suspended 5 min in the ultrasonic bath.

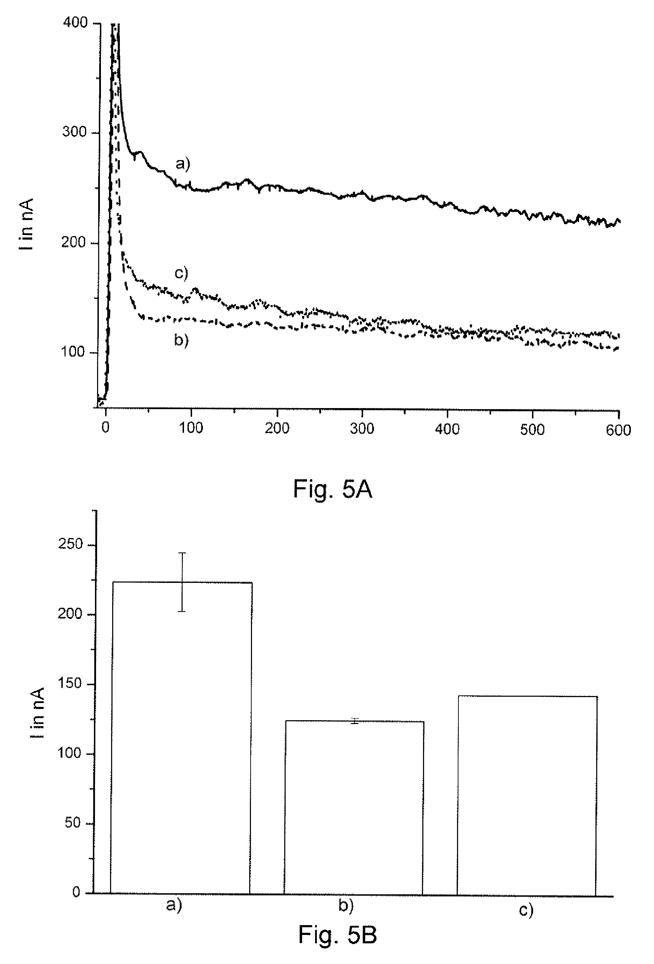

Figure 5 is showing the blood coagulation time obtained for sensors fabricated in presence of negatively charged carbon nanotubes. A) solid line (a): plasma N; dotted line (c): blood; dashed line (b): plasma P. B) current after 200 s: a) plasma N (n=4); b) plasma P (n=2); c) blood (n=1)

### Example 8: Sensors fabricated in presence of an increased amount of negatively charged carbon nanotubes.

Sensors were fabricated following example 3 (dispensing and drying) using additionally a solution of negatively charged carbon nanotubes as shown in example 7, however, an increased amount of negatively charged carbon nanotubes (solution #6) was added to the deposition solution forming the solution #5d:
■ Deposition solution #5c: 0.4 mg thrombin substrate + 1000 µl activation solution #4 (15 min in ultrasonic bath). To 510 µl of the deposition solution 6.9 mg negatively charged nanotubes and 63.2 µl resydrol AY were added. The solution was suspended 5 min in the ultrasonic bath.

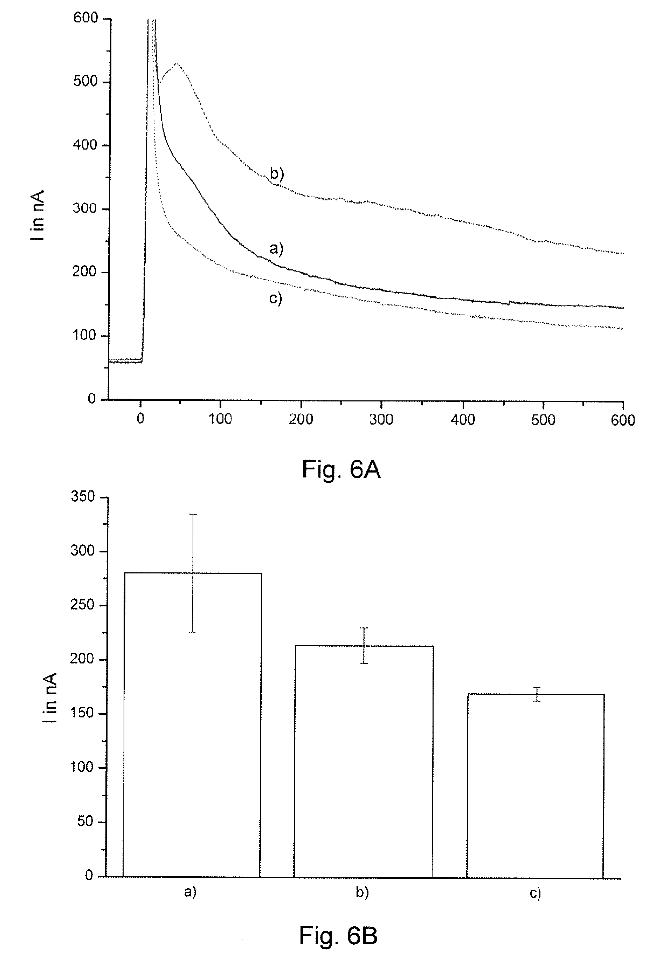

Figure 6 is showing the blood coagulation time obtained for sensors fabricated in presence of negatively charged carbon nanotubes. A) solid line (a): plasma N; pointed line (c): blood; dashed line (b): plasma P. B) current after 200 s: a) plasma N (n=4); b) plasma P (n=2); c) blood (n=1).

## Claims

1. A blood coagulation time measuring sensor comprising a surface, a resin layer applied thereto, including at least one substance with a detection function, **characterized in that** the resin layer comprises a lacquer system, wherein said lacquer system
i) is suitable for immobilization of the at least one substance with a detection function on the surface; and
ii) provides a negatively charged surface layer;
iii) and allows the replacement of biological blood coagulation promoters by non-biological chemically inert and stable compounds.

2. The blood coagulation measuring sensor according to claim 1, wherein the negatively charged surface layer is provided by the non-biological chemically inert and stable compounds.

3. The blood coagulation measuring sensor according to any one of claims 1 to 2, wherein the lacquer system is a negatively charged acrylic acid based electrodeposition polymer.

4. The blood coagulation measuring sensor according to claim 3 wherein the negatively charged acrylic-acid based electrodeposition lacquer is selected from the laquer family of Resydrol.

5. The blood coagulation measuring sensor according to claims 1 to 4, wherein the resin layer further comprises negatively charged clay materials such as bentonite, Kieselgur, or other negatively charged additives such as oxidized carbon nanotubes.

6. The blood coagulation measuring sensor according to any one of claims 1 to 5, wherein the said at least one substance with a detection function comprises at least one chemical substrate with one terminal link which can be cut off by an enzyme to give a charged compound.

7. The blood coagulation measuring sensor according to claim 6, wherein said chemical substrate reagent with one terminal link which can be cut off by an enzyme to give a charged compound is an amperogenic thrombin substrate and the enzyme is thrombin.
